# EUROPEAN PATENT APPLICATION

(11) **EP 4 338 839 A1**
(43) Date of publication of application: **20.03.2024**
(21) Application number: 22315211.7
(22) Date of filing: 14.09.2022
(51) Int. Cl.: B01L 3/00, G01N 33/493

(54) **URINE REAGENT CONTAINING DEVICE FOR URINE ANALYSIS**

(71) Applicant: Withings, 92130 Issy-les-Moulineaux (FR)
(72) Inventor: Loy, Jonas, 92130 Issy-les-Moulineaux (FR); Chan, Tung Chi, 92130 Issy-les-Moulineaux (FR); Barakat, Christelle, 92130 Issy-les-Moulineaux (FR); Donnet, Pierre-Arnaud, 92130 Issy-les-Moulineaux (FR)
(74) Representative: Withings IP

(57) **Abstract**

The present disclosure relates to a urine reagent containing devices and a method associated, the device (502) comprising: a carrier assembly (504) comprising at least a moisture-proof layer (604), the carrier assembly being arranged to define at least one open chamber (612) configured to receive a urine reagent (510); a lid assembly (506), comprising at least a moisture-proof layer, the lid assembly being arranged to cover the open chamber, the open chamber of the carrier assembly and the lid assembly defining a closed chamber (508a, 508b); a reagent in the closed chamber; wherein at least one of the lid assembly or the carrier assembly is transparent, and the closed chamber is moisture-proof.

## Description

The present disclosure relates to urine reagent containing devices for urine analysis.

### State of the art

Document WO2021/175909 discloses a point-of-care device for urine analysis. The device is to be lodged in a toilet (more precisely on a surface of a toilet bowl) and collects sample of a urine stream before performing an optical analysis. The device comprises a station and a cartridge, which is also called a rotative support, and which may be removed and replaced from the station. The cartridge contains urinary strips, for example coated or impregnated with a reagent that reacts with urine.

Each cartridge may be used for a certain number of urine analyses, after what it needs to be replaced by a new cartridge. The versatility of the device also allows to use different types of cartridges, adapted for different types of urine analysis: for instance, a cartridge could be used by athletes to check their level of nutrients while other cartridges could be used by women to assist them in managing their hormonal cycle.

The cartridge, due to its small size, its precision required and its circular shape, is challenging to manufacture, in terms of: efficiency, moisture-proofness, rate of not-good, material costs, operator time and labor cost.

In addition, the storing, shipping and use of the cartridges is challenging, as there may be several months between the manufacturing and the use of the cartridge. It is however paramount that the cartridge be in a perfect state when the user installs it and use it in the station. In particular, one of the main challenges is to maintain a high quality for the reagent throughout the cartridge's life. Whether the cartridge is stored in a humid, dry, hot, or cold environment for a few days or a few months, the reagent needs to be able to react as expected to the urine, otherwise the data generated by the device, if any at all, will be inaccurate.

At last, the above-mentioned difficulties, when combined together, create a substantial hurdle, the overcoming of which is paramount to be able to produce a high-quality cartridge. There is thus a need to develop adequate cartridges that overcome the above-mentioned challenges.

### Summary of the invention

The present disclosure relates to embodiments of urine reagent containing devices and method of manufacturing thereof that overcome the above-mentioned challenges. The urine reagent containing device may be mounted in a cartridge usable in a station for urine analysis that can be installed on a surface of a toilet bowl. The cartridge mounted on the station will be referred to as an analysis device.

The invention is defined in the appended claims.

In one embodiment, the description relates to a urine reagent containing device for a urine analysis device cartridge, the reagent containing device comprising:
- a carrier assembly made of at least two layers, one of said layers being a moisture-proof layer, the carrier assembly being arranged to define at least one open chamber;
- a lid assembly made of at least two layers, one of said layers comprising at least a moisture-proof layer, the lid assembly being arranged to cover the open chamber, the open chamber of the carrier assembly and the lid assembly defining a closed chamber; and
- a urine reagent in the closed chamber;
wherein at least one of the lid assembly or the carrier assembly is transparent, and the closed chamber is moisture-proof.

The lid assembly may be transparent. The lid assembly may be formed by a lid assembly sheet, wherein the lid assembly sheet is less than 0.5 mm thick. In an implementation, the moisture-proof layer of the lid assembly sheet is made of PCTFE.

In an implementation, the carrier assembly comprises a first liaison layer and the lid assembly sheet comprises a second liaison layer, the two liaison layers facing each other. The lid layer may comprise a sealant, to seal the liaison layer of the lid layer to the carrier assembly.

In an implementation, the first and the second liaison layers are each made of at least one of: PS, PET, PE and PVC.

The moisture-proof layer of the carrier assembly may be made of at least one of: a moisture-proof polymer, such as PCTFE, aluminum, PVC, PVDC.

In an implementation, the reagent containing device comprises a plurality of closed chambers arranged next to one another, the plurality of closed chambers being formed by a plurality of open chambers in the carrier assembly, wherein two adjacent open chambers are spaced from each other and attached to one another at a rim portion of each open chamber by a bridge portion of the carrier assembly, wherein the bridge portion and the two adjacent open chambers define therebetween a gap.

The urine reagent containing device may comprise at least 10 closed chambers and 9 gaps, in particular, more than 20 closed chambers, for example between 20 and 100, or between 50 and 100 closed chambers, or between 60 and 90 closed chambers.

In an implementation, the reagent containing device comprises a plurality of lid assemblies formed by a single lid assembly attached to at least the bridge portion of the carrier assembly, the plurality of lid assembly being arranged to cover the plurality of open chambers. The plurality may mean more than seventy, more than a hundred or more than a thousand.

The plurality of lid assemblies may be formed by a single lid assembly sheet.

In an implementation, the bridge portion is less wide than an open chamber.

In an implementation, each open chamber comprises at least one aperture, and the bridge portion and the two adjacent open chambers define therebetween a gap.

In an implementation, the open chamber includes at least one aperture and the reagent containing device further comprises a closing assembly comprising a moisture-proof layer, the closing assembly being arranged to cover the aperture, such that the open chamber of the carrier assembly, the lid assembly and the closing assembly define the closed chamber. The closing assembly is transparent.

The lid assembly may be transparent, and the closing assembly may be transparent. In an implementation, transparent means transparent to at least wavelengths comprised between 300 nm and 900 nm.

In an implementation, the closing assembly and the lid assembly are sandwitching the carrier assembly.

In an implementation, the at least one aperture of an open chamber is covered by a closing assembly freely extending at least partially over the gap, the closing assemblies being independent from each other. The closing assembly may be a closing assembly sheet, cut at the level of the gaps.

The moisture proof layer of the closing assembly may be made of PCTFE.

In an implementation, the carrier assembly only includes draft angles.

In an implementation, the carrier assembly comprises a first additional liaison layer and the closing assembly comprises a second additional liaison layer, the first and the second additional liaison layers facing each other.

The closing layer comprises a sealant, to seal the liaison layer of the closing layer to the carrier assembly.

The carrier assembly may comprise two liaison layers, sandwiching the moisture-proof layer.

The open chamber may comprise a bottom and a wall, and the aperture is defined in the bottom.

In an implementation, the carrier assembly is opaque. Opaque may mean opaque to wavelengths comprised between 300 nm and 900 nm.

In an implementation, the lid assembly is made of a material piercable with a needle with a strength of less than 5N with a beveled needle with an outer diameter of 0.5 mm.

In an implementation, the reagent containing device is flexible, so that it can be arranged in a right circular cylinder shape of at least 80% of a circle with a diameter comprised between 3 cm and 10 cm.

The description also relates to a cartridge comprising the urine reagent containing device as presented above, wherein the urine reagent containing device is arranged in a right circular cylinder shape, extending along at least 80% of a circle, with a diameter comprised between 3 cm and 10 cm.

The cartridge may further comprise a main carrier, wherein the urine reagent containing device is mounted on the main carrier. The lid assembly may be radially inwardly facing, and the main carrier may comprise a cylindrical portion and the reagent containing device may be arranged against the cylindrical portion. The carrier may be transparent.

In an implementation, the lid assembly is arranged radially innermost, and the closing assembly is arranged radially outwardly facing.

The description also relates to a urine analysis device for urine analysis comprising:
- the cartridge as presented above;
- a station, configured to be positioned on a wall of a toilet bowl, the station comprising:
   a case comprising an annular housing in which the cartridge is at least partially received and is rotatably mounted therein around a rotation axis;
   an injector, configured to pierce the lid assembly of the urine reagent containing device of the cartridge and to inject urine onto the urine reagent; and
   an analyzer, configured to emit and receive light, in relation with a closed chamber of the cartridge to detect a change of properties of the urine reagent.

The description also relates to a method to manufacture a urine reagent containing device, the method comprising:
- providing a carrier assembly sheet, comprising at least a moisture-proof layer;
- forming (e.g., embossing) the carrier assembly sheet to form the carrier assembly, to define at least one open chamber configured to receive a urine reagent;
- providing a lid assembly sheet, comprising at least a moisture-proof layer;
- placing a reagent in the open chamber;
- placing the lid assembly sheet on the carrier assembly to generate a lid assembly covering the open chamber, the open chamber of the carrier assembly and the lid assembly defining a closed chamber, wherein the closed chamber is moisture-proof, and wherein the closing assembly and/or the carrier assembly is/are transparent.

The method may further comprise:
- creating an aperture in the open chamber by punching;
- providing a closing assembly sheet, comprising at least a moisture-proof layer;
- placing the closing assembly sheet on the carrier assembly to generate a closing assembly to cover the aperture, the open chamber of the carrier assembly, the lid assembly and the closing assembly defining a closed chamber.

The method may further comprise:
- bending the urine reagent containing device;
- mounting the urine reagent containing device in the main carrier.

In an implementation, the closing assemblies are made by cutting the sheet.

In particular, the reagent containing device of the method is as presented above.

In another aspect, the description relates to a urine reagent containing device for a urine analysis device cartridge, the reagent containing device comprising:
- a carrier assembly arranged to define a plurality of open chambers spaced next to one another, wherein two adjacent open chambers are attached to one another at a rim portion of each open chamber by a bridge portion of the carrier assembly, wherein the bridge portion and two adjacent open chambers define therebetween a gap;
- a plurality of lid assembles arranged to cover the plurality of open chambers, each open chamber of the carrier assembly and each lid assembly defining a closed chamber; and
- a urine reagent in the closed chamber.

In an implementation, each open chamber includes at least one aperture, the urine reagent containing device further comprising a plurality of independent closing assemblies, wherein the at least one aperture of an open chamber is covered by a closing assembly extending at least partially over the gap, the closing assemblies being independent from one another, wherein each open chamber, each lid assembly and each closing assembly define a closed chamber, wherein the closing assemblies are transparent at least over the aperture

The closing assembly may be a closing assembly sheet, cut at the level of the gaps.

The urine reagent containing may comprise more than 10 closed chambers, in particular more than 20 closed chambers, for example between 20 and 100, or between 50 and 100, or between 60 and 90.

The urine reagent containing device may be configured to be bent in a right cylinder shape, for at least 80% of the circumference of the right cylinder shape, wherein the maximum diameter of the right cylinder shape is comprised between 3 cm and 15 cm (or even between 3 cm and 10 cm).

The lid assemblies may be formed by a single lid assembly sheet covering the at least two open chambers.

The single lid assembly sheet may be attached at least to the bridge portion of the carrier assembly.

In an implementation, the carrier assembly only includes draft angles.

In an implementation, the carrier assembly is made of at least two layers, one of said layers being a moisture-proof layer, the lid assembly is made of at least two layers, one of said layers comprising at least a moisture-proof layer, and the closed chamber is moisture proof.

In an implementation, the carrier assembly comprises a first additional liaison layer and the closing assembly comprises a second additional liaison layer, the first and the second additional liaison layers facing each other.

In an implementation, the carrier assembly comprises a first liaison layer and the lid assembly sheet comprises a second liaison layer, the two liaison layers facing each other, and wherein the carrier assembly comprises a first additional liaison layer and the closing assembly comprises a second additional liaison layer, the first and the second additional liaison layers facing each other.

The description also relates to a cartridge comprising the urine reagent containing device as presented above, wherein the urine reagent containing device is arranged in a right circular cylinder shape, extending along at least 80% of a circle, with a diameter comprised between 3 cm and 15 cm (or even between 3 cm and 10 cm).

In an implementation, the cartridge comprises a main carrier, the urine reagent containing device being mounted on the main carrier, the lid assembly being radially inwardly facing, in particular, the main carrier comprises a cylindrical portion and the reagent containing device is arranged against the cylindrical portion.

In an implementation, the lid assembly is arranged radially innermost, and the closing assembly is arranged radially outwardly facing.

The carrier may be transparent.

A urine analysis device for urine analysis comprising:
- the cartridge as presented above;
- a station, configured to be positioned on a wall of a toilet bowl, the station comprising:
   a case comprising an annular housing in which the cartridge is at least partially received and is rotatably mounted therein around a rotation axis;
   an injector, configured to pierce the lid assembly of the urine reagent containing device of the cartridge and to inject urine onto the urine reagent; and
   an analyzer, configured to emit and receive light, in relation with a closed chamber of the cartridge to detect a change of properties of the urine reagent.

The description also relates to a method to manufacture a urine reagent containing device, the method comprising:
- forming (e.g., embossing) a carrier assembly sheet to form a carrier assembly with a plurality of open chambers spaced next to one another, wherein two adjacent open chambers are attached to one another at a rim portion of each open chamber by a bridge portion of the carrier assembly, wherein the bridge portion and two adjacent open chambers define therebetween a gap;
- placing a reagent in the open chamber,
- placing a lid assembly sheet on the carrier assembly to cover the plurality of open chambers, an open chamber of the carrier assembly and the lid assembly defining a closed chamber.

The method may further comprise:
- creating at least one aperture in each chamber of the plurality of open chambers;
- placing a closing assembly sheet on the carrier assembly to cover the at least one aperture of the plurality of open chambers;
- cutting the closing assembly sheet at the level of the gaps of the carrier assembly between two adjacent open chambers, hereby creating a plurality of independent closing assemblies;
wherein an open chamber of the carrier assembly, the lid assembly and a closing assembly define a closed chamber.

The method may further comprise:
- bending the urine reagent containing device in a right circular cylinder shape of at least 80% of a circle with a diameter comprised between 3 and 10 cm, the closing assemblies facing outwardly;
- mounting the urine reagent containing device in the main carrier.

The reagent containing device may be as presented below.

### Drawings

For a better understanding of the various embodiments described in the enclosed reference is made to drawing introduced here below:
- [FIG. 1]: [Figure 1] shows the overall setup of an analysis device for urine analysis according to an embodiment, as installed on a surface of a toilet bowl;
- [FIG. 2]: [Figure 2] shows an exploded view of an embodiment of an analysis device, in which the station and the cartridge are visible;
- [FIG. 3]: [Figure 3] shows a detailed view of a cartridge according to an embodiment;
- [FIG. 4]: [Figure 4] shows a sectional view of an embodiment of a cartridge and a station, at the location of an optical analyzer of the station;
- [FIG. 5]: [Figure 5] shows different embodiments of a urine reagent containing device;
- [FIG. 6]: [Figure 6] shows other embodiments of a urine reagent containing device;
- [FIG. 7A]: [Figure 7A] shows some of the steps to manufacture a urine reagent containing device according to the embodiments of [Fig. 5];
- [FIG. 7B]: [Figure 7B] shows some further steps to manufacture a reagent containing device, included a step to mount it on the main carrier, according to the embodiments of [Fig. 5];
- [FIG. 8A]: [Figure 8A] shows some of the steps to manufacture a urine reagent containing device according to the embodiments of [Fig. 6];
- [FIG. 8B]: [Figure 8B] shows some further steps to manufacture a reagent containing device, included a step to mount it on the main carrier, according to the embodiments of [Fig. 6];
- [FIG. 9]: [Figure 9] shows an exploded view of a urine reagent containing device in an unfolded state;
- [FIG. 10]: [Figure 10] shows a partial view of a technical drawing of a urine reagent containing device in an unfolded state.

### Detailed description

The present description introduces different examples of a cartridge usable with a station as disclosed in document WO2021/175909 and WO20211175944 (publication numbers), hereafter referred to as WO'909 and WO'944. Variations of the stations are presented in any of FR2109383, FR2109384, FR2109391, FR2109392 (filing numbers).

The next paragraphs explain the overall principal of a device for urine analysis, but all the details of WO'909 and WO'933 (and also any of the above mentioned French filings) are applicable.

### OVERALL DESCRIPTION OF THE STATION AND THE CARTRIDGE

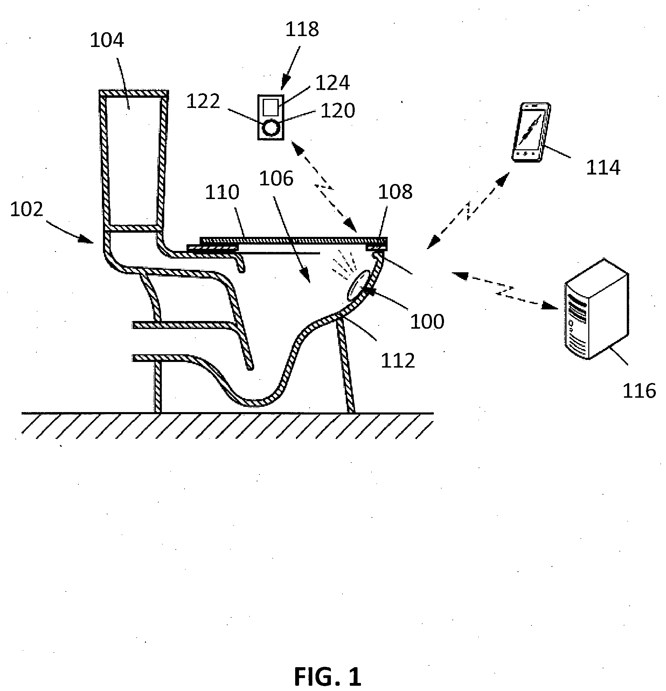

[FIG. 1] schematically illustrates an analysis device 100 (referred to as the "device 100") for urine analysis as set up in toilets 102. Toilets 102 usually comprise a water tank 104, a bowl 106, a seat 108 and a seat cover 110. The analysis device 100 is arranged in a removable manner in the toilets 102. For example, the analysis device 100 may be easily removed from the toilets to replace a cartridge and then arranged again in the toilets 102. The analysis device 100 is arranged on an internal wall 112 of the bowl 106 of the toilets. The analysis device 100 is placed so that it is usually under a urine stream from a user, such that when a user urinates (typically in a seated position), urine contacts with the analysis device 100. The analysis device 100 may communicate remotely with a remote entity, such as smartphone 114 or a server 116.

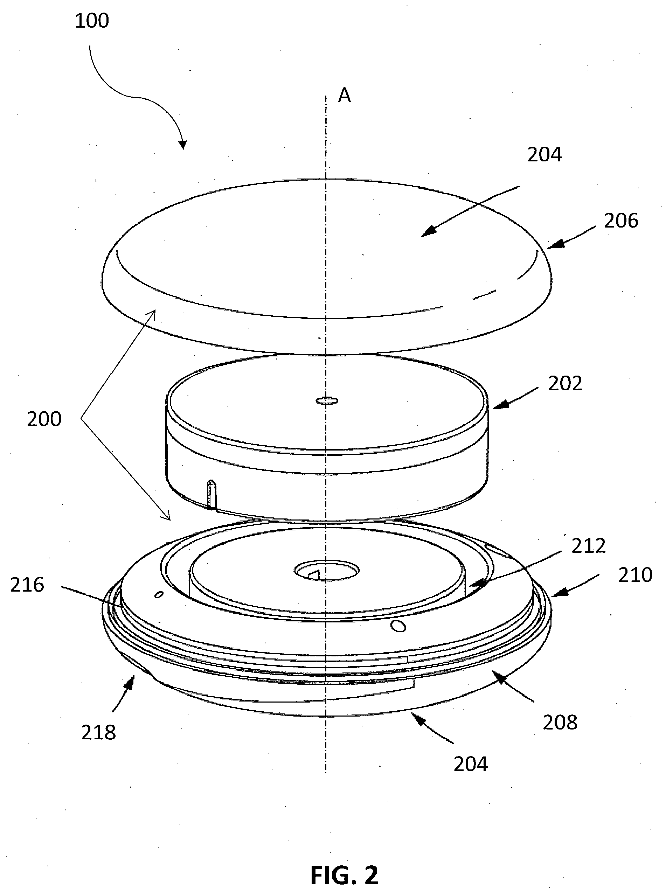

As illustrated in more detail on [FIG. 2], the analysis device 100 comprises a station 200 and a cartridge 202, mounted in a removable manner from the station 200. Station 200 may comprise a case 204 which may include two shells 206, 208. Case 204 lodges therein a urine testing assembly. Station 200 comprises an annular or ring-shaped housing 212, located inside the case 204, arranged around a rotation axis A. The annular housing 212 is configured to receive at least partially the cartridge 202 mounted in a rotatable manner around the rotation axis A (once in position in the annular housing 212). The cartridge 202 comprises a plurality of test supports which each comprise at least one urine reagent, for instance a dry reagent, the plurality of test supports being arranged along a circle or a circular arc around the rotation axis A. In an embodiment the test supports are test strips. The test supports may be enclosed, for example individually enclosed, in a chamber.

The annular housing 212 typically extends around 360° and forms a groove configured to receive at least partially the cartridge 202.

Station 200 comprises a collection opening 218, located for example on shell 208. The collection opening 218 collects urine flowing on the surface of the housing 204. A drain opening (not illustrated) is also included to drain the liquid out of the device 100.

The housing 204 may have a diameter, in a direction perpendicular to the rotation axis A, comprised between 50 and 150 mm.

The test assembly comprises a pump, an injector and an analyzer. The pump sucks urine from the collection opening 218, then the injector injects the urine on one or more test supports of the cartridge and the analyzer obtains some values of properties (e.g., physicochemical properties, such as the color) of the test supports after it has contacted the urine. In one embodiment, the analyzer is an optical analyzer configured to analyze optical properties of the test support. The injector and the cartridge may move relatively to each other so that the injector can open (e.g., pierce) the chamber, for example with a needle or needle-like device.

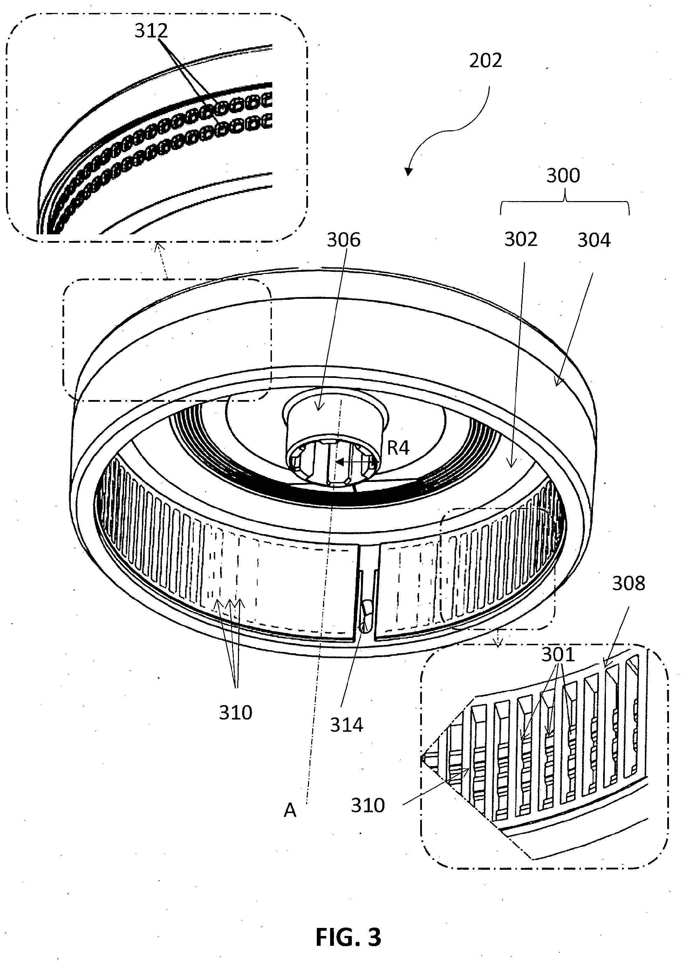

[FIG. 3] shows an exploded view of the cartridge 202. The cartridge 202 comprises test supports 301 configured to receive urine from the injector. The test support contains a urine reagent that reacts in a specific way when in contact with urine. The cartridge 202 comprises a rotative support 300, configured to be driven in rotation by the station 200. In normal use of the cartridge 202 and the device 100, the test supports 301 remain attached to the rotative support and do not move with respect to the latter.

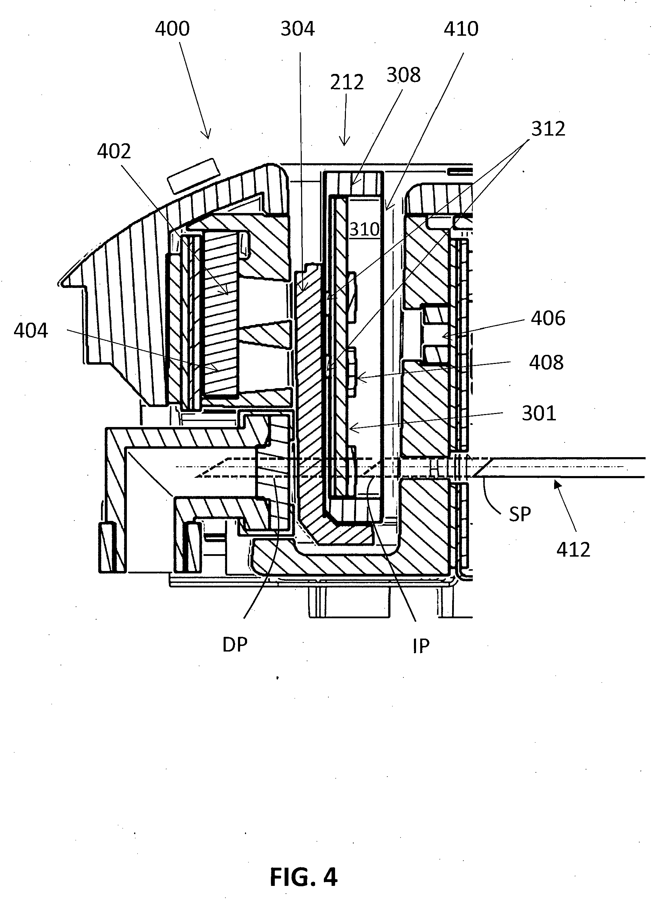

In an embodiment, the rotative support 300 has a hollow cylinder shape extending annularly around an axis which is, when the cartridge 202 is mounted in the station 200, the rotation axis A. The test support 301 may be a test strip. The rotative support 300 may comprise an annular portion 302 and a cylindrical portion 304, which extends from an outermost radial extremity of the annular portion 302. The cylindrical portion 304, when in use, is lodged inside the annular chamber 212. The test supports 301 are positioned along the cylindrical portion 304, so that they can be selectively and/or successively scrolled in front of the injector and the analyzer. For instance, the test supports 301 are part of a holder 308, which comprises several chambers 310, separated from each other along a perimeter around the axis A. To allow light to go through, the holder 308 includes at least one hole 312 per chamber 310 (represented in the upper left zoom where the rotative support is shown as transparent). The chambers 310 are all at an equal distance of the rotation axis A, so that the injector can selectively inject urine after the desired chamber is positioned at a desired location facing the injector. The injector may translate towards the chamber 310 and pierce a lid closing the chamber 310 (visible on [FIG. 4]). A drain opening 314 is provided in the rotative support 300 to allow evacuating urine from the injector to the outside of the device 100.

The annular portion 302 of the rotative support 300 remains outside of the annular chamber 212 to strengthen the cylindrical portion and/or to drive in rotation the cartridge 202. To that end, the annular portion 302 may include a mechanical coupling 306, which cooperates with a shaft of the station 200.

The dimensions relative to the cartridge 202 are disclosed in WO'909, WO'933 and the above-mentioned French applications. The maximum dimension of the device 100 transversal to the rotational axis A is less than 15 cm, even less than 10 cm. The maximum dimension of the device along the rotation axis A is less than 5 cm.

[FIG. 4] shows in more detail the interaction between the cartridge 202 and the station 200 when or after the injector is activated. The analyzer 400 comprises at least one light source 402, 404 (e.g., two) and at least one optical sensor 406. Light travels from the light source 402, 404 to the optical sensor 406 while passing through the cartridge 202 and in particular the cylindrical portion 304, the aperture 312 of the holder 308, the test support 301 and thus the reagent 408. The injector includes an injection end 412 (for example a needle), which can be moved between several positions, which are represented in dash lines in [FIG. 4]. In a standby position SP, the injection end 412 is outside the cartridge 202 (in an innermost position), so that the cartridge 202 can rotate freely in the annular housing 212; in an injection position IP, the injection end 412 has pierced the lid 410 to access the inside of the chamber 310 and may inject some urine on the test support 301; in the drain position DP, the injection end 412 is able to evacuate urine through the drain opening 314 of the rotatable support 300.

In position SP, the injector is located radially inward the annular chamber. This allows to maximize the radius of the annular chamber while minimizing the size of the station 200.

### THE REAGENT CONTAINING DEVICE

The cartridge 202 needs to provide several chambers 310 around a perimeter of the cylindrical portion 304 to accommodate the test supports 301. Those chambers 310 need to be moisture-proof and preferably regularly arranged. In addition, because *inter alia* of the cylindrical shape, the manufacturing is challenging. It will be now described different embodiments of a cartridge 202, along with the manufacturing process thereof.

### Overall description

The cartridge 202 may comprise two main parts: a reagent containing device, which includes the reagent(s), and a main carrier,. whose function is to hold the reagent containing device. In this embodiment, the reagent containing device may be manufactured on its own, before being mounted on the main carrier, which may be the rotative support 300 of [FIG. 3]. In the embodiment of [FIG. 3], the reagent containing device includes the holder 308 and the main carrier corresponds to the rotative support 300.

Alternatively, the cartridge 202 could be integral, with a reagent containing device integrated in the main carrier. This is possible when the reagent containing device is self-supporting.

In use, or when ready to be used, the reagent containing device is in a bent configuration in which the reagent containing device is arranged in a circular shape, or extends along a right circular cylinder shape on at least, for example, 80% of a circle. To simplify the manufacturing process, the reagent containing device is flexible, so that it can be manufactured in a flat configuration along a longitudinal axis and afterwards bent in a circular shape.

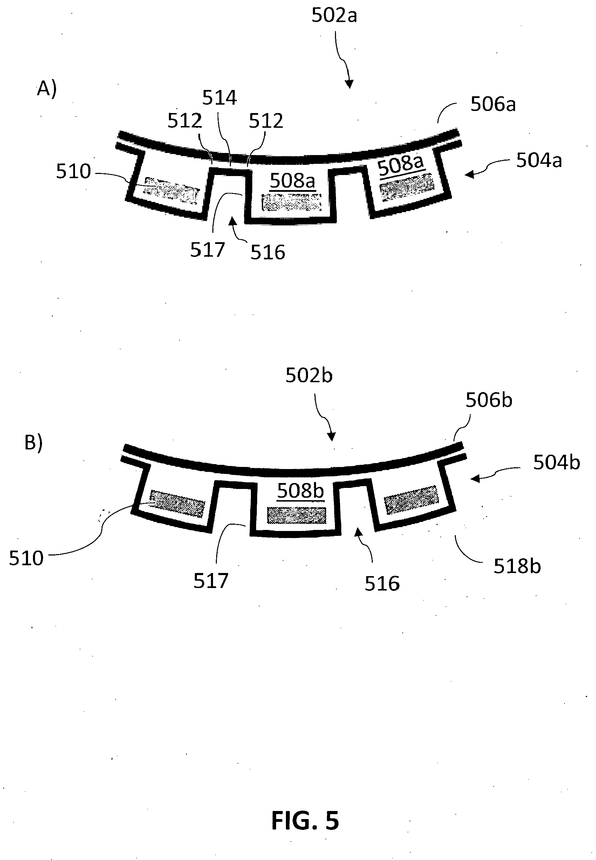

[FIG. 5] represents different variations, referenced A) and B) of the reagent containing device, coupled or not with a separate main carrier, which will be described in detail. Unless specified otherwise, any reference sign with an "a" at the end refers to variation A), "b" refers to variation B.

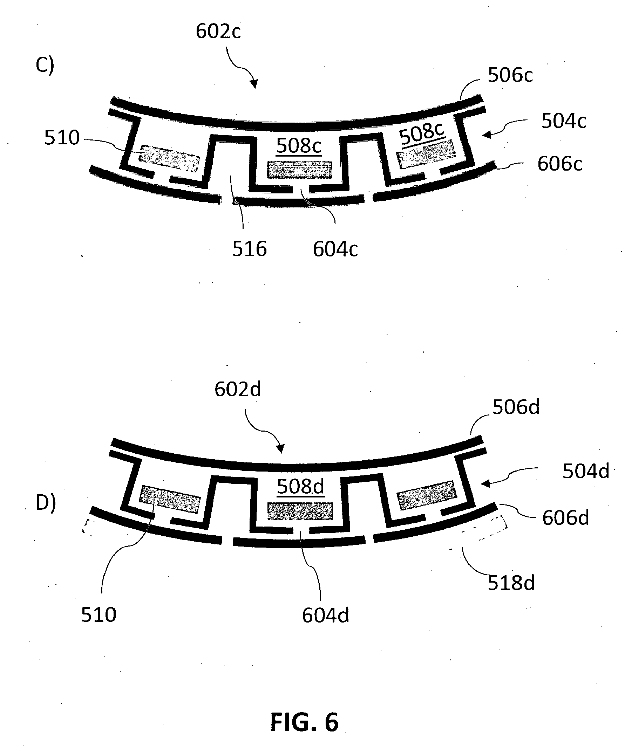

[FIG. 6] represents different variations, referenced C) and D) of the reagent containing device, coupled or not with a separate main carrier, which will be described in detail. Unless specified otherwise, any reference sign with an "c" refers to variation C) and "d" refers to variation D).

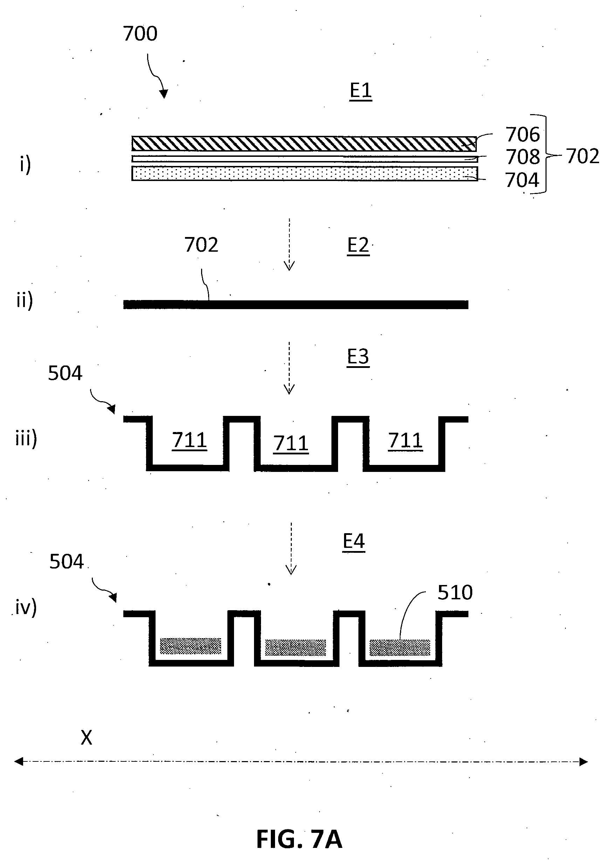

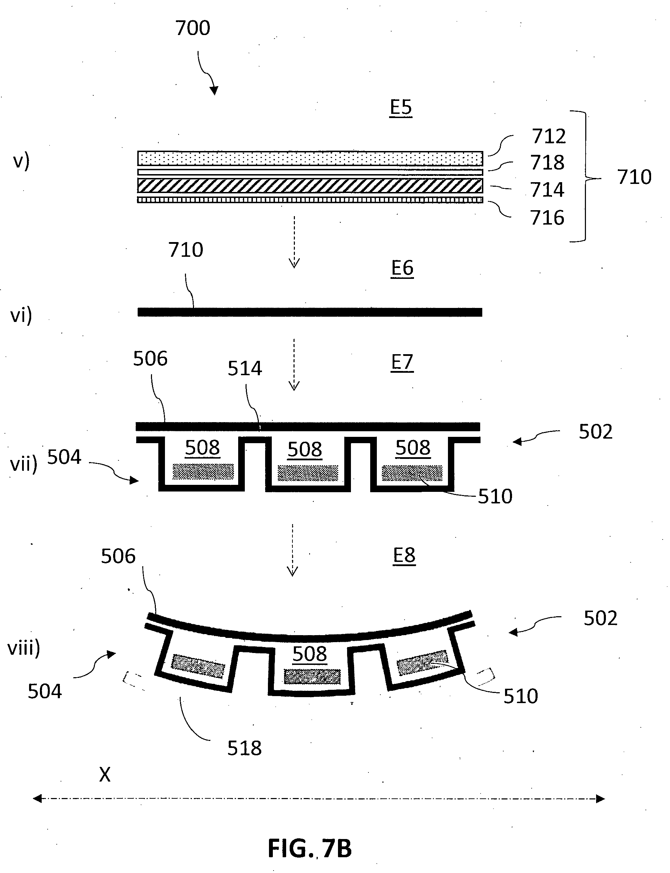

[FIG. 7A] and [FIG. 7B] illustrate a workflow representing different steps of a method to manufacture the reagent containing device according to the example B) of [FIG. 5], with additional steps to manufacture the cartridge (i.e., including the step of mounted the reagent containing device on the main carrier).

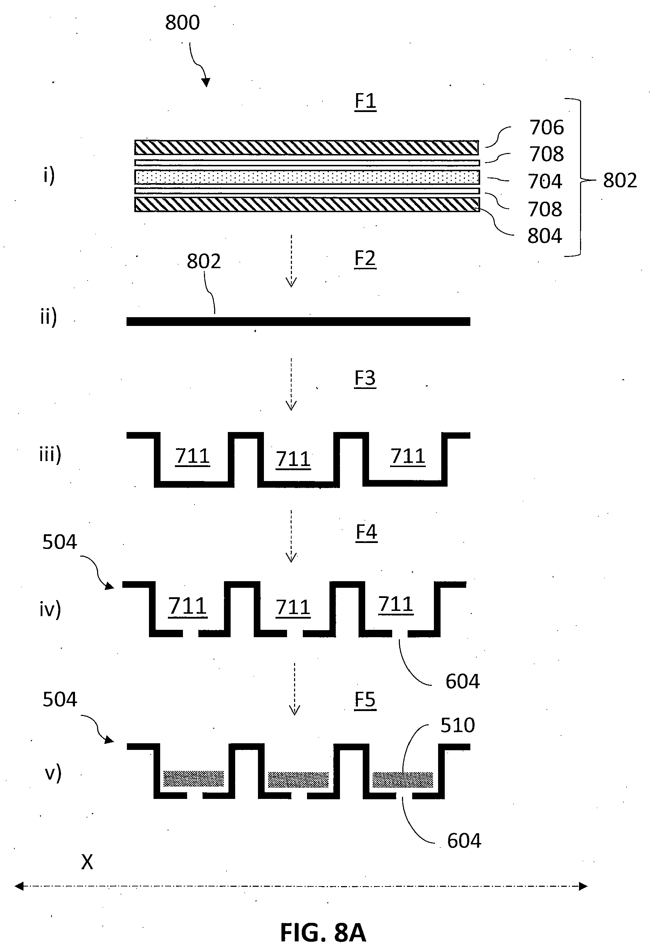

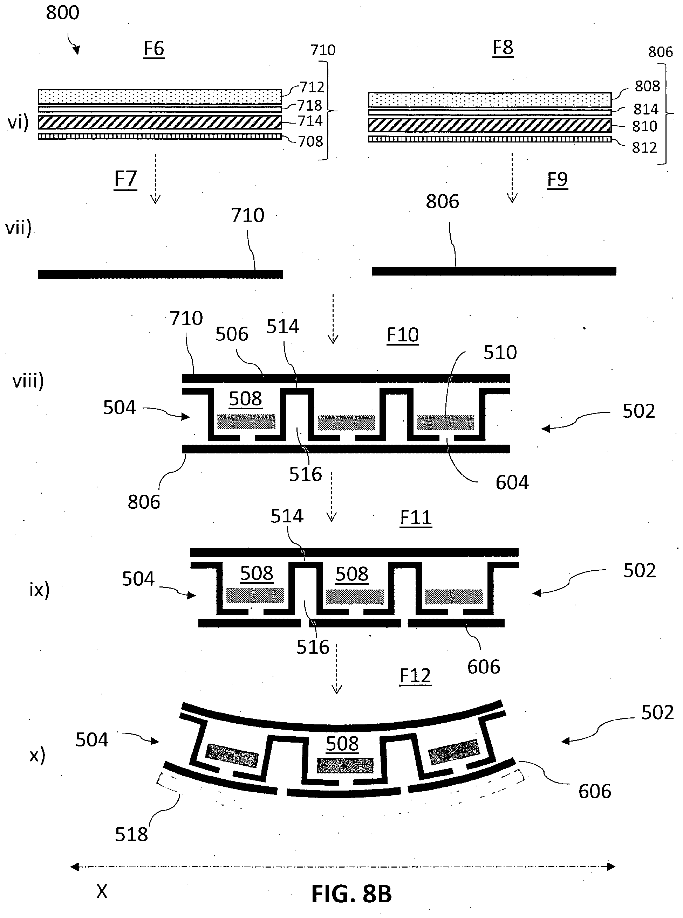

[FIG. 8A] and [FIG. 8B] illustrate a workflow representing different steps of a method to manufacture the reagent containing device according to the example D) of [FIG. 8], with additional steps to manufacture the cartridge (i.e., including the step of mounted the reagent containing device on the main carrier).

As illustrated on [FIG. 5], the reagent containing device 502a, 502b, (more generally referenced as 502) comprises a carrier assembly 504a, 504b, (more generally referenced as 504) and at least one lid assembly 506a, 506b, (more generally referenced as 506). The lid assembly 506a, 506b is radially inward in the bent configuration of the reagent containing device.

The carrier assembly 504 defines at least one open chamber (more details below and in [FIG. 7A] and [FIG. 8A] with reference 711), the function of which is to receive at least one urine reagent 510. By open, it is meant that the chamber is accessible for a machine or an operator to put the reagent 510 therein. In particular, the carrier assembly 504 comprises several open chambers, arranged next to one another, preferably in a regular manner, and spaced from each other, to accommodate as many reagents 510 as possible to have a cartridge that may run a large amount of urine analysis sessions (at least one chamber is used for one analysis). In the embodiment of the drawings, adjacent open chambers are attached to one another at a rim portion 512 of each open chambers by a bridge portion 514 of the carrier assembly 504. Under the bridge, adjacent open chambers are spaced from each other by a gap 516. Each gap 516 is open and does not form an enclosure (also referred to as an "open gap"). A gap 516 is therefore defined by one wall 517 of a closed chamber 508, the rim portion 512 adjacent to the wall 517 of said closed chamber 508, the bridge portion 514, a rim portion 512 of an adjacent closed chamber 508, the wall 517 adjacent to the rim portion 512 of said adjacent closed chamber.

In an embodiment, the geometry of the carrier assembly when laid out in a flat configuration, only includes draft angles, as visible on the figures, so that the manufacturing process of the carrier assembly may be carried out by molding with a mold moving along a single direction or by embossing along a single direction.

In an embodiment, the carrier assembly is integral (made in a single piece).

The reagent may be in a dry state, for instance incorporated on a strip, or in a liquid state.

The open chambers may be all identical in terms of dimensions.

The lid assembly 506 covers the open chamber to define a closed chamber 508, which is moisture-proof. More particularly, one lid assembly may cover one open chamber. In an embodiment, the lid assemblies are made by a single lid assembly, e.g., a lid assembly sheet, covering several consecutively adjacent open chambers.

Throughout the description, by moisture-proof, it is understood a material which has a low moisture vapor transmission rate (MVTR). By low, it is meant lower than 0.350 g/m²/day under 40°C at 75% of relative humidity (RH), and preferably lower than 0.300 g/m²/day under 40°C at 75%RH. In an embodiment, moisture proof means a MVTR value lower than 0.100 g/m2/day under 40°C at 75% RH, for instance lower than 0.065 g/m2/day under 40°C at 75% RH. In particular, the MVTR is such that the reagent can remain in the closed chamber for more than 4 months, even 6 months, or even 1 year, without losing its properties.

As the analysis is optically run, at least a wall of the closed chamber 508 (e.g., the lid assembly 506 and/or the carrier assembly 504) needs to let light therethrough: for example, said wall may be transparent to the relevant wavelength of the analyzer or the wall of the open chamber may have an aperture therein. Those wavelengths may encompass the visible spectrum, such that in an embodiment at least a wall of the closed chamber is transparent, as usually defined. For example, the transparency is defined for wavelength between 300 nm and 900 nm. Those wavelengths may in addition encompass the ultraviolet spectrum. Depending on whether optical analysis works in reflection or transmission, the closed chamber is transparent on one side or on two opposite sides. In an embodiment, the lid assembly is transparent. The skilled person will understand that by "letting the light therethrough" or "transparent", it is meant that the wall is suitable for caring out an optical analysis across the wall.

To avoid light leaks or cross-contamination from the neighboring closed chambers 508, the carrier assembly 504 may be opaque to light, in particular opaque to the relevant wavelength of the analyzer. Those wavelengths may encompass the visible spectrum, such that in an embodiment the closed chamber is opaque, as usually defined. The carrier assembly 504 may be made by an opaque material or may comprise an opaque coating.

In the examples A) and B) of [FIG. 5], the lid assembly 506a, 506b may be transparent so that the analyzer can emit lights towards the closed chamber 508a, 508b through the lid assembly 506a, 506b and obtain reflected lights from reagent in the closed chamber 508a, 508b through the lid assembly 506a, 506b again.

In an embodiment, the lid assembly 506 is transparent and the carrier assembly 504 is opaque.

Example A) is an example of self-supporting reagent containing device, without carrier, while example B) is an example of a reagent containing device with a main carrier 518b. The bending value of the drawing is also not necessarily representative of real bending values.

### The aperture and the closing assembly

In the examples C) and D) shown on [FIG. 6], other embodiments of reagent containing device 602c, 602d are represented. The reagent containing device 602c, 602d may be similar to reagent containing device 502a, 502b except for the feature which will be disclosed. The same references and same numbering of [FIG. 5] applies, with the letter "c" or "d" at the end. In those examples, the lid assembly 506c, 506d may be transparent as in the examples A) and B) of [FIG. 5]. So that the optical analysis may work in transmission, the closed chamber 508c, 508d is transparent on an opposite side of the lid assembly 506c, 506d. In that regard, to create said transparency, the open chamber may include an aperture 604c, 604d, to let the light through when the carrier assembly 504c, 504d is opaque. The aperture 604c, 604d is for example located at a bottom of the open chamber. The number of apertures 604c, 604d depends on the optical analyzer 400 (e.g., the number of light sources) and/or the number of reagents in the closed chamber. In the example of [FIG. 4], two light sources 402, 404 are used so that two apertures, configured to be facing the two light sources 402, 404 when the cartridge is in the position ready for urine injection in the closed chamber that contains the apertures, are provided.

The reagent 510 is located between the aperture 604c, 604d and the lid assembly 506c, 506d, on a light path. For moisture-proofness reasons, the reagent containing device 502c, 502d comprises a closing assembly 606c, 606d which is moisture-proof and is arranged to cover the at least one aperture 604c, 604d. In the reagent containing device 602c, 602d, the open chamber of the carrier assembly 504c, 504d, the lid assembly 506c, 506d and the closing assembly 606c, 606d define the closed chamber 508c, 508d that contains the reagent 510. To run the optical analysis, the closing assembly 606c, 606d is transparent (as described for the lid assembly 506c, 506d), at least over the aperture. In an embodiment, to simplify the manufacturing, the whole closing assembly 604c, 604d is transparent.

The closing assembly 606c, 606d may be made by a continuous sheet spreading over several successive apertures 812c, 812d or by separate smaller sheets. In this respect, each closing assembly extends at least partially over one gap 516, preferably in a free manner. More particularly, two adjacent closing assemblies 606c, 606d may extend partially over the same gap 516. When the reagent containing device 602c, 602d is lying in the flat configuration, two adjacent closing assemblies 606c, 606d may touch each other and, conversely, may get spaced from one another in the bent configuration when the reagent containing device is bent in a right cylinder shape so that the closing assemblies are facing outwardly. This may be obtained by cutting a sheet, as it will be explained later.

The carrier assembly 504 is therefore located between the closing assemblies and the lid assemblies. The lid assembly 506a, 506d is radially inward, the closing assembly is radially outward.

Example C) is an example of self-supporting reagent containing device, without carrier, while example D) is an example of a reagent containing device with a main carrier 518d. Again, the bending value of the drawing is also not necessarily representative of real bending values.

### The piercable lid assembly

The lid assembly 506 is made of a material that can be pierced by a needle or any similar shape, with a force a few Newtons (less than 30 N for a flat needle with an outer diameter inferior to 1mm for example; or even less than 20N for a flat needle with an outer diameter inferior to 0,5mm; or less than 5N for a beveled needle with an outer diameter of 0.5mm - for example around 2N). The resistance of said lid assembly 506 therefore needs to be high enough to provide a reliable and storable cartridge 202, while providing a working cartridge 202 with the test assembly, notably the injector. The combination of the lid assembly material and the thickness thereof are chosen to be piercable. The injector may be translated towards the lid assembly 506 so that the needle comes into contact with it and pierce it.

### Structure and material of the carrier assembly

The carrier assembly may be manufactured from a sheet, called a carrier assembly sheet, composed of several layers of different materials. The carrier assembly sheet may be a sheet with a thickness less than 0.5mm, even less than 0.4mm. The carrier assembly sheet is afterwards formed, notably by molding or embossing, into the appropriate shape to create the carrier assembly 504 with the open chambers.

The open chamber may be formed by a specific shape of the carrier assembly sheet, which is a formable sheet. In this regard, the material(s) composing the carrier assembly sheet are foldable or formable, for example thermoformable. Amongst those materials, thermoplastics may be used, such as polychlorotrifluoroethylene (PCTFE or PTFCE), or polyvinyldenechloride (PVDC), or some metals, such as aluminum, may be used.

As the open chamber forms a part of the closed chamber, it needs to be moisture proof. For that reason, at least one of the materials composing the carrier assembly sheet is moisture proof, that is to say with a low MVTR. PCTFE may be chosen as one of the materials of the carrier assembly 504. Aluminum may be also be chosen, as the carrier assembly 504 as such needs not be transparent (i.e. when the optical analysis works in reflection or when the open chamber includes an aperture).

In the embodiment illustrated on [FIG. 7A], items i) to iv), the carrier assembly 504 is made from a carrier assembly sheet 702 made of at least two layers: one moisture proof layer 704 (e.g., made of PCTFE or aluminum) and a first liaison layer 706. The first liaison layer 706 is aimed at interfacing the lid assembly 506 to be sealed together (as PCTFE is not easily heat-sealed and usually not recommended for that use). For example, the first liaison layer 706 is made of polystyrene (PS), polyethylene (PE), polyvinyl chloride (PVC) or polyethylene terephthalate (PET).

In the embodiment illustrated on [FIG. 8A] where the optical analysis works in transmission, a carrier assembly sheet 802 is made of at least three layers, with the moisture-proof layer 704 and the first liaison layer 706 but also with a first additional liaison layer 804 such that the moisture-proof layer 704 is sandwiched between the two liaison layers 706, 804. The first additional liaison layer 804 is aimed at interfacing the closing assembly 606 to be sealed together (as PCTFE is not suitable for sealing). The first additional liaison layer 804 may also be used to opacify the carrier assembly sheet 802, and thus the carrier assembly 504, given that PCTFE is transparent.

The liaison layers 706, 804 may be made of PS, PE, PVC, or PET.

Glue 708, as localized patches or as a layer, may be provided between any liaison layers 706, 804 and the moisture-proof layer 704 to better maintain them altogether.

### Structure and material of the lid assembly

The lid assembly 506 may be made from a lid assembly sheet 710. The lid assembly sheet 710 may be a sheet with a thickness less than 0.5 mm, or less than 0.3 mm, or even less than 0.2mm. A same lid assembly sheet may cover several open chambers to create several closed chambers 508. The lid assembly sheet 710 may comprise several layers of different materials, as it will be explained in further details, such as PCTFE and PET, PE or PS, combined or not with glue to better maintain the layers together. Those materials are transparent and are therefore appropriate for the optical analysis.

As the lid assembly 506 forms a part of the closed chamber 508, it needs to be moisture-proof. For that reason, at least one of the materials composing the lid assembly 506 is moisture proof, with a low MVTR; PCTFE may be chosen as one of the materials of lid assembly 506.

In the embodiment illustrated on [FIG. 7B], items v) and vi), and in the embodiment illustrated on [FIG. 8B], items vi) and vii), the lid assembly is made from a lid assembly sheet 710 made of at least two layers: one moisture-proof layer 712 (e.g., of PCTFE) and a second liaison layer 714. The second liaison layer 714 is aimed at interfacing the carrier assembly 504 to be sealed together (as PCTFE is not easily heat-sealed and usually not recommended for that use). In that regard, the second liaison layer 714 is facing the carrier assembly 504, and more specifically the first liaison layer 706 of the carrier assembly 504. For example, the second liaison layer 714 is made of PS, PE, PVC, or PET. A sealant 716 (e.g., a layer of sealant) may be used between the second liaison layer 714 of the lid assembly sheet 710 and the first liaison layer 706 of the carrier assembly 504.

Glue 718, as localized patches or a layer, may be provided between the second liaison layers 714 and the moisture-proof layer 712.

### Structure and material of the closing assembly

Referring to [Fig. 6] and [Fig. 8B], in an embodiment, the closing assembly 606 may be made from a closing assembly sheet 806. The closing assembly sheet 806 is similar to the lid assembly sheet 710, in terms of material and property, although different material may be used between the closing assembly sheet 806 and the lid assembly sheet 710, providing they comply with the moisture-proofness requirements and the sealing requirements. In addition, the closing assembly 606 does not need to be pierced and can therefore be more resistant (e.g., thicker; e.g. with more resistant materials).

The closing assembly sheet 806 is a sheet with a thickness less than 0.5 mm, or less than 0.3 mm, or even less than 0.2mm. A same closing assembly sheet may cover several apertures 604 to create several closed chambers 508. The closing assembly sheet 806 may be made of several layers of different materials, as it will be explained in further details, such as PCTFE and PET (polyethylene terephthalate) or PE or PS, combined or not with glue to better maintain the layers together. Those materials are transparent and are therefore appropriate for the optical analysis.

As the closing assembly 606 forms a part of the closed chamber 508, it needs to be moisture-proof. For that reason, at least one of the materials composing the closing assembly 606 is moisture proof, with a low MVTR; PCTFE may be chosen as one of the materials of closing assembly 514.

In the embodiment illustrated on [FIG. 8B], item vi), the closing assembly 606 is made from the closing assembly sheet 806 made of at least two layers: one moisture-proof layer 808 (e.g., made of PCTFE) and a second additional liaison layer 810. The second additional liaison layer 810 is aimed at interfacing the carrier assembly 504 to be sealed together (as PCTFE is not easily heat-sealed and usually not recommended for that use). In that regard, the second additional liaison layer 810 is facing the carrier assembly 504, and more specifically the first additional liaison layer 804. For example, the second additional liaison layer 810 is made of PET, PE (polyethylene) or PS. A sealant 812 (e.g., a layer of sealant) may be used between the second additional liaison layer 810 of the closing assembly sheet 806 and the first additional liaison layer 804 of the carrier assembly 504.

Glue 814, as localized patches or a layer, may be provided between the second additional liaison layers 810 and the moisture-proof layer 808.

### The urine reagent

As already mentioned, the urine reagent 510 may be incorporated on a strip (e.g., coated, impregnated, etc.), such as a paper strip. The strip, when the cartridge is in use in the station 200, extend parallel to the rotation axis A. All the strips together, in their respective closed chambers, are arranged along on a cylindrical shape around the rotation axis A. This means that the strips are at a same distance from the rotation axis A, to enable the injection process by the injector.

The urine reagents that may be used are: antibodies (anti-HCG beta for example), antigens (hormones), indicator dyes, ionophore, etc.

### The main carrier

Referring again to [FIG. 5], [FIG. 6], [FIG. 7B] item viii), [FIG. 8B] item x), the reagent containing device 502 may be mounted on the main carrier 518b, 518d (referred to as 518). The main carrier corresponds to the rotative support 300 of FIG. 3: the main carrier has a hollow cylinder shape extending annularly around an axis which is, when the cartridge 202 is mounted in the station 200, the rotation axis A. The main carrier may comprise an annular portion 302 and a cylindrical portion 304, which extends from an outermost radial extremity of the annular portion 302. The cylindrical portion 304, when in use, is lodged inside the annular chamber 212. When the reagent containing device 502 is mounted on the main carrier 518, the closed chambers 508 are all an equal distance of the rotation axis A.

The main carrier 518b, 518d is at least optically transparent at the location of the apertures 812c, 812d, so that light can go through. As the analyzer 400 is part of the station, light is emitted from the light source 402, 404 that is radially outside the cylindrical portion 304 of the cartridge 202, while the optical sensor 406 is radially inside the cylindrical portion 304. In an embodiment, the main carrier 518b, 518d is entirely transparent. However, when the optical analysis works in reflection on the side of the lid assembly, the main carrier 518b, 518d may be opaque.

The main carrier may be made of plastic.

### Number of closed chambers

Each reagent containing device 502 may comprise more than 10 closed chambers, in particular, more than 20 closed chambers, for example between 20 and 100, or between 50 and 100 closed chambers, or between 60 and 90 closed chambers. As described above, the closed chambers are arranged next to one another, thanks to the open chambers of the carrier assembly. The number of gaps 516 is usually the number of closed chambers minus one.

### Flexibility

In some embodiment, the reagent containing device 502 is flexible so that it can be manufactured in the flat configuration on a flat surface and then bent into the bent configuration to form a cartridge ready for use (e.g., bent to be mounted in the main carrier 518b, 518d, which presents a cylindrical shape). This allows for a much easier and cost-efficient manufacturing process, that will be disclosed hereinafter. By flexible, it is means that the reagent containing device 502 may be arranged in a right circular cylinder shape around a main axis, which corresponds to the rotational axis A when the cartridge is arranged in the device. The right circular cylinder shape may extend along at least 80% of a circle with a diameter comprised between 3cm and 15cm, or even between 3cm and 10cm, for example between 5cm and 8cm, or even between 6cm and 6.5cm.

The plurality of open chambers, the gaps 516 between adjacent closed chambers 508, and the localized sealing parts (as the sealing intervenes at the interface between the carrier assembly 504 and the lid assembly) contributes to generate a flexible reagent containing device, besides the flexibility of the materials itself. In the embodiments of [FIG. 6], flexibility is further achieved by having a plurality of independent closing assemblies 606c, 606d, thereby avoiding the resistance that a single closing assembly covering several apertures of several open chamber would have. Thus, the configuration disclosed in the present description is particularly to be in a bent configuration.

### Manufacturing process

The manufacturing process will enlighten some of the technical considerations underlying some specific embodiments of the reagent containing device. [FIG.7A], [FIG. 7B] and [FIG.8A], [FIG. 8B] illustrate two workflows 700 and 800 representing different steps of a method to manufacture the reagent containing device according to the example B) of [FIG. 5] and the example D) of [FIG. 6].

Workflow 700 will now be described in detail.

Referring to [FIG.7A], at step E1, the moisture-proof layer 704 (e.g., made of PCTFE) and the first liaison layer 706 (e.g., made of PS) are provided.

At step E2, the carrier assembly sheet 702 is manufactured by laminating the moisture-proof layer 604 with the first liaison layer 706 as disclosed previously. Glue 708 may be placed therebetween to strengthen the carrier assembly sheet 702. Placing machines and laminating machines may be used in step M2.

At step E3, the carrier assembly sheet 702 is deformed (e.g., embossed) to generate the carrier assembly 504 with the open chambers 711. Due to the deforming process, the carrier assembly 504 may only have draft angle along a deforming direction, in the flat configuration. The material of the inside of open chamber 711 is thus the material of the first liaison layer 706 (e.g., made of PS). An embossing device may be used in step E3. The carrier assembly 504 may be made from a carrier assembly sheet long enough to include thousands of open chambers 710. In addition to the low MVTR, PCTFE has efficient thermoforming properties and is therefore advantageously used in the carrier assembly. However, other thermoplastics or thermopolymers may be used.

At step E4, the reagent 510 is placed in the open chamber 711. For instance, the reagent 510 is part of a test strip, such as paper strip or paper-like strip; the test strip is thus placed inside the open chamber 711. A precision tooling may be used in step M4.

Referring to [FIG.7B], at step E5, the moisture-proof layer 712 (e.g., made of PCTFE) and a second liaison layer 714 are provided (e.g., made of PS).

At step E6, the lid assembly sheet 710 is manufactured by superposing and laminating the moisture-proof layer 712 on the second liaison layer 714. Glue 718 may be placed therebetween to strengthen the lid assembly sheet 710; sealant 716 may be used to improve thermo-sealing.

At step E7, the lid assembly sheet 710 is placed on the carrier assembly 504 to covet the open chamber 710 to create the closed chamber 508, and more specifically a plurality of open chambers to create a plurality of closed chambers. The lid assembly sheet 710 and the carrier assembly 504 are heat sealed together. In that regard, the layer of sealant 716 may be used. As explained above, the first and second liaison layers 706, 714 are facing each other, as the moisture-proof layers 704, 712 as such may not be easily heat sealed. The open chamber 711 containing the reagent is now a moisture-proof closed chamber 508. The interface between the carrier assembly 504 and the lid assembly sheet 710 includes the bridge portion 514 between two adjacent open chambers 711. The lid assembly sheet 710 has thus created the lid assembly 506. A heat-sealing machine may be used in step E7.

At step E8, which is optional depending on the embodiment of the reagent containing device, the reagent containing device 502 is mounted on the main carrier 516, thereby taking a circular shape. The reagent containing device 502 may be attached onto the carrier with some glue or adhesive, or may simply be fitted therein.

Workflow 800 will now be described in detail.

Referring to [FIG.8A], at step F1, the moisture-proof layer 704 (e.g., made of PCTFE), the first liaison layer 706 (e.g., made of PS) and the first additional liaison layer 804 are provided.

At step F2, the carrier assembly sheet 802 is manufactured by sandwiching and laminating the moisture-proof layer 604 between the first liaison layer 706 and the first additional liaison layer 804, as disclosed previously. Glue 708 may be placed therebetween to strengthen the carrier assembly sheet 802. Placing machines and laminating machines may be used in step M2.

At step F3, the carrier assembly sheet 602 is deformed (e.g., embossed) to generate the carrier assembly 504 with the open chambers 711. Due to the deforming process, the carrier assembly 504 may only have draft angle along a deforming direction. The material of the inside of open chamber 711 is thus the material of the first liaison layer 706 (e.g., made of PS). An embossing device may be used in step F3. The carrier assembly 504 may be made from a sheet that includes thousands of open chambers 711. In addition to the low MVTR, PCTFE has efficient thermoforming properties and is therefore advantageously used in the carrier assembly. However, other thermoplastics or thermopolymers may be used.

At step F4, at least one aperture 604 is created in the bottom part of the open chamber 711, for example by punching. In this regard, a punching device with a punch may be used in step F4. Punching is cost-efficient and quick. However, the apertures 604 may be created differently, by laser cutting or drilling.

At step F5, the reagent 510 is placed in the open chamber 711. For instance, the reagent 510 is part of a test strip, such as paper strip or paper-like strip; the test strip is thus placed inside the open chamber 711. A precision tooling may be used in step M4.

Referring to [FIG.8A], at step F6, the moisture-proof layer 712 (e.g., made of PCTFE) and a second liaison layer 714 are provided (e.g., made of PS).

At step F7, the lid assembly sheet 710 is manufactured by superposing and laminating the . moisture-proof layer 712 on the second liaison layer 714. Glue 718 may be placed therebetween to strengthen the lid assembly sheet 710; sealant 716 may be used to improve thermos-sealing.

At step F8, the moisture-proof layer 808 (e.g., made of PCTFE) and a second additional liaison layer 810 are provided (e.g., made of PS).

At step F9, the closing assembly sheet 806 is manufactured by superposing and laminating the moisture-proof layer 808 on the second additional liaison layer 810. Glue 814 may be placed therebetween to strengthen the lid assembly sheet 806; sealant 812 may be used to improve thermos-sealing.

Steps F6, F7 and F8, F9 may be carried out independently.

At step F10, the lid assembly sheet 710 is placed on the carrier assembly 504 to cover the open chamber 711 to create the closed chamber 508, and more specifically a plurality of open chambers to create a plurality of closed chambers. The lid assembly sheet 710 and the carrier assembly 504 are heat-sealed together. In that regard, the layer of sealant 708 may be used. As explained above, the first and second liaison layers 706, 714 are facing each other, as the moisture-proof layers 704, 714 as such may not be easily heat sealed. The interface between the carrier assembly 504 and the lid assembly sheet 710 includes the bridge portion 514 between two adjacent open chambers 711. In addition, at step F10 (or in another step), the closing assembly sheet 806 is placed on the carrier assembly 504 to cover the aperture 604. In that regard, the layer of sealant 812 may be used. More specifically, the closing assembly sheet 806 cover several apertures 604 of several respective open chamber. As explained above, the first additional liaison layer and the second additional liaison layers 804, 810 are facing each other, as the moisture-proof layers 704, 808 as such may not be easily heat sealed. The interface between the carrier assembly 504 and the closing assembly sheet 806 includes the portions of the carrier assembly 504 around the apertures 604. The lid assembly sheet 710 has thus created the lid assembly 506. A heat-sealing machine may be used in step F10.

After step F10, the closing assembly sheet 806 is a continuous layer covering the apertures 604 of a plurality of open chambers. In particular, the closing assembly sheet 806 may extend over the gaps 516 of the carrier assembly 504, between two adjacent closed chambers 508. As explained above, the closing assembly sheet 806 is deemed to be the radially outermost element of the reagent containing device after it is bent in a circular shape. The continuous closing assembly sheet 806 could therefore oppose to the bending of the reagent containing device.

In an embodiment, not illustrated, the closing assembly sheet 806 is sufficiently extensible so that the reagent containing device may be bent in a right circular cylinder shape. However, another embodiment will be disclosed in detail.

At step F11, the closing assembly sheet 806 is cut at the level of the gap 516 of the carrier assembly 504 between two adjacent chambers. This cutting step is notably permitted by the fact that the closing assembly sheet 806 is away from the carrier assembly 504 at the gap, in particular away from the bridge portion 514 between two adjacent closed chambers 508. It is therefore possible to cut the closing assembly sheet 806 without cutting the carrier assembly 504, thereby maintaining the structural integrity of the carrier assembly 504. One cut at the gap 516 between two adjacent chambers 508 may be provided, as illustrated. This step F11 creates a plurality of closing assemblies 606, made by several independent pieces of the former closing assembly sheet 806. Due to the step of one-cutting, the independent pieces may each extend at least partially over the gap 516, in a free manner (as they are free to move because they are not heat-sealed on the carrier assembly 504). A cutting machine may be used at step F11. The cutting machine may comprise a plurality of cutters, so that in one batch a plurality of single cuts between adjacent chambers 508 are created. For instance, the cutting machine may comprise between 10 and 100 cutters arranged next to one another, appropriately spaced.

At step F12, which is optional depending on the embodiment of the reagent containing device, the reagent containing device 502 is mounted on the main carrier 516, thereby taking a circular shape. Bending the reagent containing device is simplified by the structure of the carrier assembly (the gap and bridge portion, wherein the bridge is radially internal while the gap is radially external), and by the cut closing assemblies 606. The reagent containing device 502 may be attached onto the carrier with some glue or adhesive, or may simply be fitted therein.

### Dimensions and strip-shape

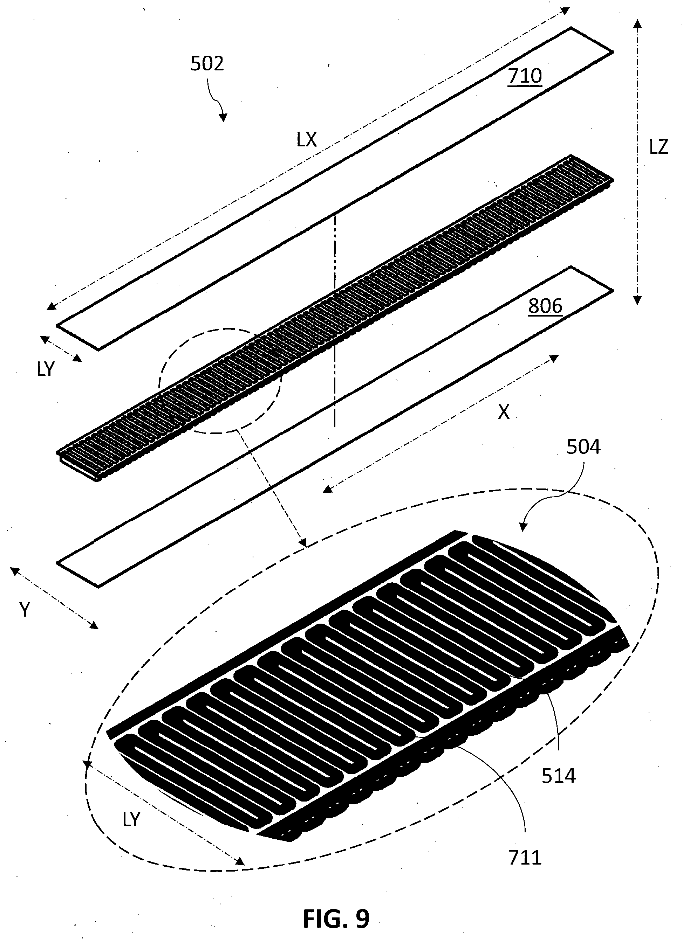

[FIG. 9] illustrates an exploded view of the reagent containing device 502 in the flat configuration comprising the carrier assembly 504 (reagent not visible) sandwiched between the lid assembly sheet 710 and the closing assembly sheet 806, with a carrier assembly 504 lying flat. They are all shaped as strips extending along a longitudinal axis X. The length LX (i.e. the dimension along the longitudinal axis X) of the reagent containing device 502 in the flat configuration may be between 150mm and 200mm. The width LY (i.e. the dimension across the longitudinal axis X) of the reagent containing device 502 in the flat configuration may be between 10mm and 20mm, for example between 13mm and 16mm. The thickness LZ of the reagent containing device 502 in the flat configuration (i.e. the last dimension across the longitudinal axis) may be between 1mm and 3mm, for example between 2.0mm and 2.5mm. A reagent containing device, when laid out on a flat surface, has the LX, LY and LZ dimensions.

As visible on [FIG. 9], the open chamber(s) 711 extend along the width of the strip, perpendicular to the longitudinal axis X. To minimize the overall size of the cartridge 202, the open chamber 711 extends along almost the entire width of the carrier assembly 504, for example more that 80%. The remaining portions of the carrier assembly 504, at the extremity of the open chamber 612 are used to seal the lid assembly sheet 710 onto the carrier assembly 504 to generate the moisture-proof chamber 508. An open chamber 711 may be between 10mm and 15mm long (depending on the width of the strip) and between 1.0mm and 1.5mm wide (the width of the open chamber being along the longitudinal axis X). The depth of the open chamber is less than the overall thickness of the carrier assembly, due to the thickness of the carrier assembly sheet 602 after forming.

The gaps 516, being defined by two adjacent closed chambers, extends along the entire width of the carrier assembly to enable the above-mentioned flexibility

The plurality of open chambers 711 are positioned parallel to one other, as close as possible to each other to maximize *in fine* the number of close chambers in one cartridge. All the open chambers 711 preferably have identical dimensions. For example, the distance along the longitudinal axis X between two adjacent open chambers 612, i.e., the distance along the bridge portion 514 (referred to as the sealing distance) is between 0.5 and 2.0 mm. The plurality of open chambers 612 are aligned along the longitudinal axis X. In other words, the respective extremities on each side of the plurality of open chambers 612 respective to the transversal axis Y are aligned along the longitudinal axis X. As already explained, the portion of the carrier assembly 504 therebetween is used to seal the lid assembly sheet 710 onto the carrier assembly 504 to create the moisture-proof chamber 508. Along the longitudinal axis X, the ratio of the cumulated width of the open chambers to the length LX is equal or more than 50%, which means that the ratio of the cumulated sealing distance (or bridge portion) to the length LX is strictly less than 50%. Said differently, along the longitudinal axis X, the distance between two adjacent open chambers is equal or less than the width of an open chamber 612. Each reagent containing device 502 may comprise more . than 10 closed chambers, in particular, more than 20 closed chambers, for example between 20 and 100, or between 50 and 100 closed chambers, or between 60 and 90 closed chambers, each chamber comprising at least one reagent. This allows for an optimized ratio of the number of reagents to the overall size of the reagent containing device. In another embodiment, along the longitudinal axis X, the distance between two adjacent open chambers is more than the width of an open chamber 612. This enables a greater area for sealing the lid assembly 506 onto the carrier assembly 504, although it constitutes a tradeoff with the number of closed chambers *in fine.*

Thanks to the ratios disclosed, the structure of the carrier assembly 504 the materials that may be slightly extensible, and the independent closing assemblies, bending the reagent containing device into the bent configuration is easily achievable without damaging the structure of the reagent containing device 502, and, in particular, while keeping its moisture proofness. Mounting it on the main carrier 1001 is therefore simplified.

To optimize the manufacturing process, the reagent containing device 502 and the component thereof may be manufactured using long strips extending along the longitudinal axis X, such that at the end of step E7 or F11, the outcome being several reagent containing devices arranged next to each other on a single strip. Before carrying step E8 or F12, a step of cutting the strip to the appropriate length to obtain a reagent containing device is performed, based on the number of closed chambers desired for the reagent containing device. For instance, the sheets 702, 710, 802, 806 of [FIG. 7A], item ii), of [FIG. 7B], item vi), of [FIG. 8A], item ii), of [FIG. 8B], items vii) may be provided as long strips.

By long, it is meant longer than the LX length of a single reagent containing device 504. By longer, it is meant at least ten times, even a hundred times, or even a thousand times longer than the LX length. In terms of closed chambers, the strip may contain more than five hundreds, or even a thousand, or ten thousand closed chambers. Each closed chamber contains at least one reagent that may be the same in every chamber or different between chambers. The width of the long strip may be the LY width, so that no longitudinal cut of the layers is required to manufacture the reagent containing device 502.

Specific thicknesses of the layer will be given as examples. The thickness of the liaison layers 706, 804 (e.g., made of PS, PE, PVC or PET) of the carrier assembly sheet 702, 802 may be between 100µm and 150µm, and the thickness of the moisture-proof layer 704 (e.g., made of PCTFE) may be between 30µm and 70µm. The color of the carrier assembly sheet 702, 802 may be black. The thickness of the liaison layer 714, 810 (e.g., made of PS, PE, PVC or PET) of the lid assembly sheet 710 and/or of the closing assembly sheet 806 may be between 30µm and 70µm and the thickness of the moisture-proof layer 706 may be between 10µm and 20µm. The global thickness of the lid assembly sheet 710 and/or the closing assembly sheet 806 may be less than 0.1mm.

### Example values

In the specific embodiment using PS, PE and PCTFE as disclosed above, the relative humidity (RH) at 30°C is less than 20% after a year (with the reagent containing device manufactured at 15% RH at 25°C). The transparency of the material is more than 90% in the visible spectrum and, in an embodiment, in the UV spectrum as well. For example, the strength to pierce the lid assembly is less than 20N for a flat needle with an outer diameter of 0.5 mm; or less than 5N for a beveled needle with an outer diameter of 0.5mm - for example around 2N). The choice of the material also ensures that the reagent containing device remains chemically inert in more extreme conditions, such as 90 RH at 35°C.

### Technical drawing

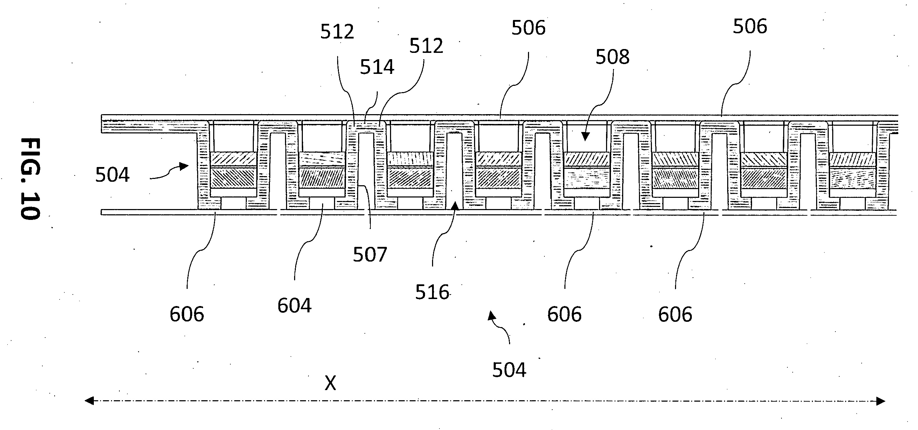

[Fig. 10] shows a partial view of a technical drawing according to an embodiment. The reagent containing device of [Fig. 10] is in the flat configuration and not yet arranged in a circular shape. Typically, [Fig. 10] illustrates the outcome of step F11.The reagent 510 here is incorporated in a paper strip comprising several layers, as described in WO'909 and WO'944.

## Claims

1. A urine reagent containing device (504) for a urine analysis device cartridge, the reagent containing device comprising:
- a carrier assembly (504) arranged to define a plurality of open chambers (711) spaced next to one another, wherein two adjacent open chambers are attached to one another at a rim portion (512) of each open chamber by a bridge portion (514) of the carrier assembly (504), wherein the bridge portion (514) and two adjacent open chambers (512) define therebetween a gap (516);
- a plurality of lid assembles (506) arranged to cover the plurality of open chambers, each open chamber of the carrier assembly (504) and each lid assembly defining a closed chamber (508); and
- a urine reagent (510) in the closed chamber (508).

2. The urine reagent containing device of claim 1, wherein each open chamber (711) includes at least one aperture (604), the urine reagent containing device further comprising a plurality of independent closing assemblies (508), wherein the at least one aperture (604) of an open chamber is covered by a closing assembly (508) extending at least partially over the gap (516), the closing assemblies (508) being independent from one another,
wherein each open chamber, each lid assembly (506) and each closing assembly (514) define a closed chamber (508).

3. The urine reagent containing device of claim 2, wherein the closing assembly (514) is a closing assembly sheet (806), cut at the level of the gaps.

4. The urine reagent containing device of any of claims 1-3, comprising more than 10 closed chambers, in particular more than 20 closed chambers, for example between 20 and 100, or between 50 and 100, or between 60 and 90.

5. The urine reagent containing device of any of claims 1-4, wherein the urine reagent containing device is configured to be bent in a right cylinder shape, for at least 80% of the circumference of the right cylinder shape, wherein the maximum diameter of the right cylinder shape is comprised between 3 cm and 10 cm.

6. The urine reagent containing device of any of claims 1-5, wherein the lid assemblies (506) are formed by a single lid assembly sheet covering the at least two open chambers, wherein the single lid assembly sheet is attached at least to the bridge portion of the carrier assembly.

7. The urine reagent containing device of any of claim 1-6, wherein the carrier assembly only includes draft angles.

8. The urine reagent containing device of any of claims 1-7, wherein:
- the carrier assembly (504) is made of at least two layers, one of said layers being a moisture-proof layer (604);
- the lid assembly (506) is made of at least two layers, one of said layers comprising at least a moisture-proof layer (704); and
- the closed chamber is moisture proof.

9. The urine reagent containing device of claim 8, wherein the carrier assembly (504) comprises a first additional liaison layer (608) and the closing assembly (514) comprises a second additional liaison layer (1006), the first and the second additional liaison layers (608, 1004) facing each other.

10. The urine reagent containing device of any of claims 1 to 9, wherein the carrier assembly (504) comprises a first liaison layer (706) and the lid assembly sheet (710) comprises a second liaison layer (714), the two liaison layers (706, 714) facing each other, and wherein the carrier assembly (504) comprises a first additional liaison layer (608) and the closing assembly (514) comprises a second additional liaison layer (1006), the first and the second additional liaison layers (608, 1004) facing each other.

11. A cartridge (202) comprising the urine reagent containing device of any of claims 1-10, wherein the urine reagent containing device is arranged in a right circular cylinder shape, extending along at least 80% of a circle, with a diameter comprised between 3 and 10 cm.

12. The cartridge of claim 11, wherein the lid assembly is arranged radially innermost, and the closing assembly is arranged radially outwardly facing.

13. A urine analysis device (100) for urine analysis comprising:
- the cartridge (202) of any of claims 11-12;
- a station (200), configured to be positioned on a wall of a toilet bowl, the station comprising:
a case (204) comprising an annular housing (212) in which the cartridge is at least partially received and is rotatably mounted therein around a rotation axis (A);
an injector (412), configured to pierce the lid assembly of the urine reagent containing device of the cartridge and to inject urine onto the urine reagent; and
an analyzer (400), configured to emit and receive light, in relation with a closed chamber of the cartridge to detect a change of properties of the urine reagent.

14. A method to manufacture a urine reagent containing device, the method comprising:
- forming a carrier assembly sheet (602) to form a carrier assembly (504) with a plurality of open chambers spaced next to one another, wherein two adjacent open chambers are attached to one another at a rim portion (512) of each open chamber by a bridge portion (514) of the carrier assembly (504), wherein the bridge portion (514) and two adjacent open chambers (512) define therebetween a gap (516);
- placing (F5) a reagent in the open chamber;
- placing a lid assembly sheet (710) on the carrier assembly (504) to cover the plurality of open chambers (612), an open chamber of the carrier assembly and the lid assembly defining a closed chamber (508).

15. The method of claim 14, further comprising:
- creating (F4) at least one aperture (812) in each chamber of the plurality of open chambers (612);
- placing (F10) a closing assembly sheet (1001) on the carrier assembly (504) to cover the at least one aperture of the plurality of open chambers;
- cutting (F11) the closing assembly sheet at the level of the gaps (516) of the carrier assembly between two adjacent open chambers, hereby creating a plurality of independent closing assemblies;
wherein an open chamber of the carrier assembly, the lid assembly and a closing assembly define a closed chamber (508).

16. A urine reagent containing device (502) for a urine analysis device cartridge (202), the reagent containing device (502) comprising:
- a carrier assembly (504) made of at least two layers, one of said layers being a moisture-proof layer (604), the carrier assembly being arranged to define at least one open chamber (612);
- a lid assembly (506) made of at least two layers, one of said layers comprising at least a moisture-proof layer (704), the lid assembly being arranged to cover the open chamber, the open chamber of the carrier assembly and the lid assembly defining a closed chamber (508); and
- a urine reagent in the closed chamber;
wherein at least one of the lid assembly or the carrier assembly is transparent, and the closed chamber is moisture-proof.

17. The urine reagent containing device of claim 16, comprising a plurality of closed chambers (508) arranged next to one another, the plurality of closed chambers being formed by a plurality of open chambers (612) in the carrier assembly (504), wherein two adjacent open chambers are spaced from each other and attached to one another at a rim portion (512) of each open chamber by a bridge portion (514) of the carrier assembly, wherein the bridge portion (514) and the two adjacent open chambers (512) define therebetween a gap (516).

18. The urine reagent containing device of claim 17, further comprising a plurality of lid assemblies (506) formed by a single lid assembly attached to at least the bridge portion of the carrier assembly, the plurality of lid assembly being arranged to cover the plurality of open chambers.

19. The urine reagent containing device of any of claims 17-18, wherein the bridge portion is less wide than an open chamber (612).

20. The urine reagent containing device of any claims 16-19, wherein each open chamber comprises at least one aperture, and wherein the bridge portion and the two adjacent open chambers define therebetween a gap.

21. The urine reagent containing device of any of claims 16-20, wherein the open chamber (711) includes at least one aperture (604), the reagent containing device further comprising a closing assembly (514) comprising a moisture-proof layer (1004), the closing assembly being arranged to cover the aperture (604), such that the open chamber (711) of the carrier assembly (504), the lid assembly (506) and the closing assembly (606) define the closed chamber (508);
wherein the closing assembly (606) is transparent.

22. The urine reagent containing device of claims 20 and 21, wherein the at least one aperture of an open chamber is covered by a closing assembly freely extending at least partially over the gap, the closing assemblies being independent from each other.

23. The urine reagent containing device of claim 22, wherein the closing assembly (514) is a closing assembly sheet (1001), cut at the level of the gaps.

24. The urine reagent containing device of any of claim 16-23, wherein the carrier assembly only includes draft angles.

25. The urine reagent containing device of any of claims 16-23, wherein the reagent containing device is flexible, so that it can be arranged in a right circular cylinder shape of at least 80% of a circle with a diameter comprised between 3 cm and 10 cm.

26. A cartridge (202) comprising the urine reagent containing device of any of claims 16-25, wherein the urine reagent containing device is arranged in a right circular cylinder shape, extending along at least 80% of a circle, with a diameter comprised between 3 and 10 cm.

27. The cartridge (202) of claim 26, further comprising a main carrier (518), the urine reagent containing device being mounted on the main carrier (518), the lid assembly being radially inwardly facing, the main carrier (516) comprising a cylindrical portion (304) and the reagent containing device being arranged against the cylindrical portion (304).

28. The cartridge of any of claims 26-27, wherein the lid assembly is arranged radially innermost, and the closing assembly is arranged radially outwardly facing.

29. A urine analysis device (100) for urine analysis comprising:
- the cartridge (202) of any of claims 26-27;
- a station (200), configured to be positioned on a wall of a toilet bowl, the station comprising:
a case (204) comprising an annular housing (212) in which the cartridge is at least partially received and is rotatably mounted therein around a rotation axis (A);
an injector (412), configured to pierce the lid assembly of the urine reagent containing device of the cartridge and to inject urine onto the urine reagent; and
an analyzer (400), configured to emit and receive light, in relation with a closed chamber of the cartridge to detect a change of properties of the urine reagent.

30. A method to manufacture a urine reagent containing device, the method comprising:
- providing a carrier assembly sheet (602), comprising at least a moisture-proof layer (604);
- forming the carrier assembly sheet (602) to form the carrier assembly (504), to define at least one open chamber (612) configured to receive a urine reagent (510);
- providing a lid assembly sheet (710), comprising at least a moisture-proof layer (706);
- placing a reagent in the open chamber;
- placing the lid assembly sheet (710) on the carrier assembly (504) to generate a lid assembly (506) covering the open chamber (612), the open chamber of the carrier assembly and the lid assembly defining a closed chamber (508), wherein the closed chamber is moisture-proof, and wherein the closing assembly and/or the carrier assembly is/are transparent.

31. The method of claim 30, further comprising:
- creating an aperture in the open chamber) by punching;
- providing a closing assembly sheet, comprising at least a moisture-proof layer;
- placing the closing assembly sheet on the carrier assembly to generate a closing assembly to cover the aperture, the open chamber of the carrier assembly, the lid assembly and the closing assembly defining a closed chamber (508).

32. The method of any of claims 30-31, further comprising:
- bending the urine reagent containing device;
- mounting the urine reagent containing device in the main carrier.

33. The method of any of claims 30-32, wherein forming includes embossing.

34. The method of any of claims 30-33, wherein the closing assemblies are made by cutting the sheet.

35. The method of any of claims 30-34, wherein the reagent containing device is according to any of claims 1 to 10.
